# EUROPEAN PATENT APPLICATION

(11) **EP 2 992 900 A1**
(43) Date of publication of application: **09.03.2016**
(21) Application number: 15175245.8
(22) Date of filing: 15.10.2010
(51) Int. Cl.: A61K 39/395, A61P 29/00, C07K 16/24

(54) **ANTIBODY THERAPEUTICS WITH LOCAL ACTIVITY IN THE DIGESTIVE TRACT**

(30) Priority: 15.10.2009 US 251996 P
(62) Divisional of application: 10824210.8
(71) Applicant: Avaxia Biologics, Inc., Burlington, MA 01803 (US)
(72) Inventor: FOX, Barbara S., Wayland, MA 01778 (US); STAFFORD, Douglas C., Fitchburg, WI 53711 (US)
(74) Representative: Lee, Nicholas John

(57) **Abstract**

This invention provides methods and compositions of antibody therapeutics that are therapeutically effective in the digestive tract or below the mucosal barrier of the digestive tract but do not deliver levels of antibody to the systemic circulation that have been shown to be necessary for clinical benefit following systemic administration of antibody. This invention further describes therapeutic compositions of antibody therapeutics that are therapeutically effective in the digestive tract or below the mucosal barrier of the digestive tract but do no deliver levels of antibody to the systemic circulation that have been associated with adverse events and systemic immunosuppression following systemic administration of antibody.

## Description

### BACKGROUND OF THE INVENTION

Antibody therapeutics have proven to be valuable pharmaceuticals. However, their use is frequently limited by side effects that result from the activity of the antibody in sites throughout the body. For example, antibodies specific for TNF (Remicade, Humira, Cimzia) are effective in the treatment of inflammatory bowel disease. However, their use is associated with an increased risk of malignancy and an increased risk of serious infection (Bongartz et al., 2006, JAMA, 295, 2275-85), likely due to systemic immunosuppression. Therefore, there is a need to generate methods and pharmaceutical compositions of antibody therapeutics that are able to direct the antibody to the location where clinical benefit will be greatest while minimizing the activity of the antibody in other sites. More particularly, there is a need to generate methods and pharmaceutical compositions of antibody therapeutics that are able to direct the antibody to the digestive tract for the treatment of diseases of the digestive tract, while minimizing the activity of the antibody in other sites.

The use of antibody therapeutics is also frequently limited by the immunogenicity of the administered antibody. The induction of antibodies against the therapeutic agent is associated with loss of activity and with the potential for adverse infusion reactions. Immunogenicity is seen most clearly in cases where the antibody is derived from a non-human species. However, the presence of human anti-human antibody responses (HAHA) has also been described and is the cause of significant clinical concern (Ritter et al, 2001, Cancer Res, 61, 6851-9; Tracey et al., 2008, Pharmacol Ther, 117, 244-79). Therefore, there is a need to generate methods and pharmaceutical compositions of antibody therapeutics that are able to minimize immunogenicity while maintaining efficacy.

In the treatment of inflammatory bowel disease, patients treated with systemically administered anti-TNF antibodies frequently become non-responsive to antibody therapy due to the induction of neutralizing antibodies (Tracey et al., 2008, Pharmacol Ther, 117, 244-79). There is a need to generate methods and pharmaceutical compositions that can be used to treat patients who have become unresponsive to existing anti-TNF antibody therapeutics.

### SUMMARY OF THE INVENTION

This invention provides methods and compositions of antibody therapeutics that are therapeutically effective in the digestive tract or below the mucosal barrier of the digestive tract but do not deliver levels of antibody to the systemic circulation that have been shown to be necessary for clinical benefit following systemic administration of antibody. This invention further describes therapeutic compositions of antibody therapeutics that are therapeutically effective in the digestive tract or below the mucosal barrier of the digestive tract but do not deliver levels of antibody to the systemic circulation that have been associated with adverse events and systemic immunosuppression following systemic administration of antibody.

### BRIEF DESCRIPTION OF THE FIGURES

Figure 1 is a graph showing the endoscopy score after oral administration of various dosages of anti-TNF antibody, AVX-470, as compared to control, in a mouse model of inflammatory bowel disease.
Figure 2 is a graph showing the standard curve of an ELISA assay for the detection of serum bovine immunoglobulin antibody levels.
Figure 3 is a micrograph of stained sections of hamster cheek pouch showing that bovine immunoglobulin could be detected in the irradiated left buccal cheek pouch (panel A), but was only found on the exterior face of the non-irradiated right cheek pouch (panel B).
Figure 4 is a graph showing the survival (as a %) of mice with GI acute radiation syndrome after treatment with oral anti-TNF antibody.
Figure 5 is micrograph of stained sections of the jejunal lamina propria of mice with GI acute radiation syndrome after administration of oral anti-TNF antibody.

### DETAILED DESCRIPTION OF THE INVENTION

For the purposes of the invention, the "digestive tract" consists of the mouth, pharynx, esophagus, stomach, small intestine (duodenum, jejunum, ileum), large intestine (cecum, colon, rectum) and anus. For the purposes of the invention, the "oral cavity" is understood to include the mouth, the pharynx and the esophagus. For the purposes of the invention, the "gastrointestinal tract", or "GI tract" is understood to include the stomach, small intestine (duodenum, jejunum, ileum), large intestine (cecum, colon, rectum) and anus.

The serum levels of anti-TNF antibody, for example, associated with clinical benefit are concentrations above 0.5 ug/ml (Nestorov, 2005, J Rheumatol Suppl, 74, 13-8: Tracey et al., 2008, Pharmacol Ther, 117, 244-79). While the serum levels of anti-TNF antibody associated with adverse events is not precisely known, it is thought to be greater than the levels needed for clinical benefit (Nestorov, 2005, J Rheumatol Suppl, 74, 13-8). Thus, as compared to existing parenteral TNF antagonists the therapeutic compositions and methods of the present invention are associated with reduced systemic immunosuppression, reduced systemic distribution, reduced immunogenicity, reduced tachyphylaxis (whether caused by the induction of neutralizing antibodies or by another mechanism) and reduced immediate side effects (e.g. infusion reactions).

The therapeutic antibody compositions of the present invention minimize the activity of the therapeutic antibody outside of the digestive tract and further minimize the induction of a neutralizing immune response against the therapeutic antibody. The therapeutic compositions are antibodies that are delivered topically to the luminal face of the digestive tract. Antibodies of the invention may be administered topically to the digestive tract by, for example, oral administration, rectal administration and includes all forms of administration to the oral cavity such as by buccal, mucoadhesive films and the like. The antibodies may cross the mucosal barrier of the digestive tract to enter the submucosal space to interact with their targets, but do not enter the systemic circulation. This invention includes the use of antibodies directed at biological targets expressed on or near the luminal surface of the digestive tract as well as below the mucosal barrier such as on the basal side of the epithelium, targets expressed in the submucosa, target expressed in the lateral intercellular space, and targets expressed in the lamina propria.

In one embodiment of this invention, antibodies of the invention cross the mucosal barrier as a result of pre-existing damage to the mucosal barrier. In healthy subjects, the digestive tract is normally relatively impermeable to proteins applied to the luminal face. However, in many disease states, the digestive tract displays increased permeability (McGuckin et al., 2009, Inflamm Bowel Dis, 15, 100-13). This increased permeability permits antibody applied to the luminal face of the gut to cross the mucosal barrier (Worledge et al., 2000, Dig Dis Sci, 45, 2298-2305).

In one embodiment of this invention, antibodies of the invention cross the mucosal barrier as a result of specific aspects of the formulation that facilitate the transit of antibody across the mucosal barrier. Permeation enhancers are available, including chitosan, poly-L-arginine and Carbopol,

Patients with Crohn's disease and ulcerative colitis collectively referred to in the art as inflammatory bowel disease are frequently treated with antibodies directed against the inflammatory cytokine TNF. In these patients, there is a correlation between the clinical benefit achieved by the antibody and the trough serum concentrations of the administered anti-TNF antibody (Seow et al., Gut., 1/2010; 59(1): 49-54) (Karmiris et al., 11/2009, Gastroenterology; 137(5): 1628-40). These data indicate that a certain concentration of antibody must be obtained in the serum in order for the antibody to be effective in treating digestive inflammation.

However, the present inventors have unexpectedly discovered that local treatment of digestive inflammation using antibodies in accordance with the invention is therapeutically effective. Such local or topical application may be achieved by topical administration of an antibody such as by oral or rectal administration. The term "topical application" to the digestive tract is defined as local and/or surface administration to the oral cavity, delivery by oral or rectal administration to the digestive tract, or administration by any other route that brings the antibody in contact with the luminal aspect of the digestive tract.

Therefore, it is an object of this invention to treat digestive inflammation by administering a therapeutically effective amount of the antibodies of the invention to the digestive tract while maintaining levels of antibody in the serum below those previously believed to be needed for clinical efficacy when the antibody is given by injection. In accordance with the invention, serum levels of antibody administered in accordance with the invention are less than about 1 µg/ml preferably less than about 500 ng/ml, preferably less than about 150 ug/ml, and preferably less than about 50 ng/ml as measured using standard assays as are known in the art.

Measurements of antibody levels in serum can be accomplished using standard assays as are known in the prior art. One assay is a radioimmunoassay (RIA) that detects binding of TNF to antibody (Svenson et al., Rheumatology 2007 46:1828-1834). Briefly, 1% patient serum is added to 5,000 counts per min/0.1 ml of ¹²⁵I-TNF. After incubation for 2 hours at 4°C, free and antibody-bound tracer are separated by addition of a rabbit antibody specific for the antibody species used as the therapeutic antibody. For detection of bovine anti-TNF antibody in patient serum, rabbit antibody specific for bovine immunoglobulin (heavy + light chain) is used. For detection of infliximab (a mouse-human IgG1:κ chimeric antibody) in patient serum, rabbit antibody specific for human Fcγ is used. Rabbit antibody is added in an amount capable of precipitating >95% of available therapeutic antibody. After another 2 hours, 2.5 ml cold assay buffer is added and bound and free ¹²⁵I-TNF are separated by centrifugation at 4000g for 10 min at 4°C. Radioactivity in the pellet activity is measured using a gamma counter. The therapeutic antibody is used as a reference to construct a standard curve. For infliximab, the detection limit of the assay is 0.4 ug/ml of whole serum (Bendtzen, Arthritis and Rheumatism, Vol. 54, No. 12, December 2006, pp 3782-3789, 2006).

Another assay that can be used to detect antibody levels in serum is the enzyme linked immunosorbent assay (ELISA) (Wolbink, Ann Rheum Dis 2005; 64:704-707). Briefly, flat bottomed microtiter plates are coated overnight at room temperature with a mouse monoclonal antibody directed against TNF (2 ug/ml in 100 ul per well). Recombinant TNF (0.1 ug/ml in a 100 ul per well) in HPE buffer is added for 1 hour. After washing with phosphate buffered saline/0.2% Tween, patients' serum samples are added in different dilutions in HPE buffer and incubated for 2 hours at room temperature. Plates are washed with phosphate buffered saline/0.2% Tween, then incubated with horseradish peroxidase (HRP) conjugated to an antibody specific for the antibody species used as the therapeutic antibody. For detection of bovine anti-TNF antibody in patient serum, sheep anti-bovine IgG (h+l) is used. For detection of infliximab, monoclonal anti human IgG is used. HRP-labeled antibodies are added in 100 ml HPE buffer for 1 hour at room temperature. Subsequently, after washing, tetramethylbenzidine is added. The reaction is stopped with 2 M H₂SO₄. Absorption at 450 nm is then determined. Results are related to a titration curve of the therapeutic antibody (for example, bovine anti-TNF antibody or infliximab) in each plate. The lowest level of detection for infliximab is 0.2 ug/ml (Wolbink, page 704, *supra*).

These two assays used to measure levels of the therapeutic antibody in serum are based on detection of the antibody's ability to bind to the target antigen (e.g., TNF). An alternative assay measures the presence of the therapeutic antibody itself. This would not be preferred when the therapeutic antibody is a chimeric or humanized monoclonal antibody, because it is difficult to differentiate the therapeutic antibody from antibody normally present in the patient. However, this method may be used to detect a non-human antibody therapeutic (see Example 3). For a polyclonal antibody, only a portion of the administered antibody will be specific for and bind to the target antigen. In the context of this invention, the important antibody concentration to measure is the concentration of therapeutic antibody specific for the target antigen, as this is the component with biological activity. Therefore, when serum levels of the administered antibody are quantified through the detection of the antibody itself, experiments must be performed to determine the percentage of the administered antibody that is specific for the target antigen. A description of a method that can be used to determine the percentage is provided in Example 1.

Peak plasma levels for infliximab have been reported at 118 ug/ml and for adalimumab at 4.7 ug/ml (Tracey, 2008 *supra*). The peak plasma levels obtained will be dependent on the route of administration and the dosing schedule. Clinically effective serum concentrations for injected anti-TNF antibodies are 0.8-1.4 ug/ml (Tracey et al., 2008, Pharmacol Ther, 117, 244-79).

In one embodiment of the invention, antibodies specific for cytokines that regulate inflammation, including but not limited to TNF, TNF-kappa, Ifn-gamma, IL-1 beta, IL-2, IL-6, IL-12, IL-13, IL-15, IL-17, IL-18, IL-21, IL-23, IL27, IL-32, IL-33 and IL-35 are applied topically to the digestive tract of a patient with increased permeability of the digestive tract to prevent the development of frank ulceration or inflammation due to chemotherapy or radiation therapy.

In one embodiment of the invention, antibodies specific for cytokines that regulate inflammation, including but not limited to TNF, TNF-kappa, Ifn-gamma, IL-1 beta, IL-2, IL-6, IL-12, IL-13, IL-15, IL-17, IL-18, IL-21, IL-23, IL27, IL-32, IL-33 and IL-35 are applied topically to the digestive tract of a patient with increased permeability of the digestive tract to prevent the development gastrointestinal acute radiation syndrome due to exposure to high levels of radiation.

In one embodiment of the invention, antibodies specific for inflammatory cytokines, including but not limited to TNF, TNF-kappa, Ifn-gamma, IL-1 beta, IL-2, IL-6, IL-12, IL-13, IL-15, IL-17, IL-18, IL-21, IL-23, IL27, IL-32, IL-33 and IL-35 are applied topically to the digestive tract of a patient with increased permeability of the digestive tract to prevent the development of frank ulceration or inflammation due to autoimmune disease, including inflammatory bowel disease.

In one embodiment of the invention, antibodies specific for inflammatory cytokines, including but not limited to TNF, TNF-kappa, Ifn-gamma, IL-1 beta, IL-2, IL-6, IL-12, IL-13, IL-15, IL-17, IL-18, IL-21, IL-23, IL27, IL-32, IL-33 and IL-35 are applied topically to the digestive tract of a patient with increased permeability of the digestive tract to treat celiac disease.

In one embodiment of the invention, antibodies specific for Toll-like receptors that are expressed on the basolateral face of mucosal epithelial cells are applied as a therapeutic agent to the mucosa of the digestive tract of a patient with an intestinal inflammatory disease.

In one embodiment of the invention, antibodies specific for inflammatory cytokines, including but not limited to TNF, TNF-kappa, Ifn-gamma, IL-1 beta, IL-2, IL-6, IL-12, IL-13, IL-15, IL-17, IL-18, IL-21, IL-23, IL27, IL-32, IL-33 and IL-35 are applied as a therapeutic agent to the digestive tract of a patient with irritable bowel syndrome.

In one embodiment of the invention, antibodies directed at enteric neurotransmitters or their receptors or transporters expressed below the mucosal barrier of the digestive tract, including receptors for serotonin that are expressed in the gut (5-HT1A, 5-HT1B/B, 5-HT2A, 5-HT2B, 5-HT3, 5-HT4, 5-HT7, 5-HT1P) are used as pharmaceutical agents in patients with increased permeability of the digestive tract.

In one embodiment of the invention, antibodies directed at peptides that regulate food intake or the receptors for such peptides are used as pharmaceutical agents in patients with increased permeability of the digestive tract. Such peptides include but are not limited to CCK, GLP1, GIP, oxyntomodulin, PYY3-36, enterostatin, APOAIV, PP, amylin, GRP and NMB, gastric leptin and ghrelin (Cummings and Overduin, 2007, J Clin Invest, 117, 13-23).

In one embodiment of the invention, antibodies directed at epidermal growth factor receptor on colorectal cancer cells are used as therapeutic agents in patients with increased permeability of the digestive tract.

In one embodiment of this invention, antibodies delivered to the digestive tract that are specific for soluble cytokines reduce levels of those cytokines in the digestive tract but not in the systemic circulation. Levels of cytokine can be determined by direct measurement of the cytokine or by analysis of a surrogate marker that responds to the cytokine. In one aspect of this invention, antibodies delivered to the digestive tract that are specific for soluble cytokines reduce levels of those cytokines in the digestive tract and in the systemic circulation.

In one embodiment of this invention, antibodies delivered to the digestive tract that have clinical benefit do not induce an antibody response to the administered antibody that is sufficient to inhibit the response to subsequent doses of the antibody or to cause an injurious response to subsequent doses of the antibody.

In one embodiment of this invention, the lack of an induced antibody response is seen following maintenance therapy. In one embodiment of this invention, the lack of an induced antibody response is seen following episodic dosing. The antibody response can be measured by direct measurement of antibody specific for the therapeutic antibody or by assessment of the physiological response to repeated doses of the therapeutic antibody.

In one preferred embodiment, fewer than 2% of patients develop antibodies to the therapeutic antibodies of the invention after exposure to 3 or more doses of therapeutic antibody. In one preferred embodiment, administration of 3 or more doses of the antibody in accordance with the invention does not lower the efficacy of the antibody. In accordance with the invention, the efficacy of the antibody is not diminished after administration of 1, 2, 3 or more doses over a period of about 1 month from the date of first administration of the antibody and preferably over a period of about 6 months from the date of the first administration of the antibody, more preferably over a period of about 1 year from first administration of the antibody, and even more preferably over a period of about 10 years from first administration of the antibody.

Measurements of the antibody response to the therapeutic antibody (TA) can be accomplished using standard assays as are known in the prior art. One assay is an RIA (Svenson 2007 *supra*). The therapeutic antibody is labeled with ¹²⁵I using chloramine-T, purified by molecular size chromatography, and tested to ensure that it retains binding to appropriate antibodies known to be specific for the therapeutic antibody. ¹²⁵I-labeled TA is co-incubated for 18 hour at 4°C with patient serum (1% or lower). The anti-TA activity is assayed by binding of ¹²⁵I-TA to an affinity matrix containing a second antibody capable of specifically binding >80% of the AA in 10% serum. For infliximab, this second antibody is anti-human-λ-light chain. For bovine anti-TNF antibody, this second antibody is anti-bovine IgG (h + 1). The mean background binding is calculated and two times the background value is used to discriminate between positive and negative samples.

Another assay that can be used to detect antibody responses to the therapeutic antibody is a solid phase RIA (Svenson, 2007 *supra*). The TA (8 ug/ml) is bound to microtiter plates followed by blockade of non-occupied sites with human serum albumin. Patient sera are added in duplicate, using 10% or lower levels, at 100 ul/well. After overnight incubation at 4°C, the wells are washed with cold assay buffer and 0.1 ml of 6000 cpm of ¹²⁵I-labcled TA are added per well. After 3 hours at 4°C, the wells are washed and the bound cpm are determined.

It is well known to those skilled in the art that assays for the antibody response to the therapeutic antibody will need to be adapted to the specific situation and the methods and considerations are well understood (Gorovits, The AAPS Journal, Vol. 11, No. 1, March 2009). Of particular importance is the potential presence of preexisting antibody responses to the therapeutic antibody that may complicate statistical analysis of the data collected to calculate the assay cut-off parameter, used to distinguish a positive and negative sample. For the assay of antibody responses to bovine antibody therapeutics, it is preferred to use a direct comparison of the pretreatment and post-treatment sample results for a given patient. A patient is defined as having an induced antibody response against the therapeutic antibody if the levels of anti-TA antibody significantly increase during the course of treatment with the therapeutic antibody. For therapeutic antibodies where preexisting antibody responses are not detected, a patient is defined as having an induced antibody response against the therapeutic antibody if the antibody response is greater than two-fold above background.

Antibody responses to the therapeutic antibody were seen during maintenance therapy for inflammatory bowel disease in 2.8% - 3.7% of patients treated with adalimumab, in 5% - 18% of patients treated with infliximab and in 8% - 12.3% of patients treated with certolizumab (Cassinotti, Inflamm Bowel Dis, Vol. 15(8), August 2009). Episodic dosing resulted in higher frequencies of antibody responses to the therapeutic antibody, with 36% - 61% of patients receiving infliximab generating antibody responses.

In addition to the digestive tract, this invention may also be applied to other tissues with mucosal barriers, including the urogenital system and the respiratory system. Further, this invention may also be applied to other tissues with an epithelial system, including the eye and the skin.

In accordance with the methods of the invention, the mucosal barrier of the digestive tract may be breached or compromised through mechanical trauma, including but not limited to dental and oral wounds, esophageal wounds, or surgically induced trauma due to partial gut resection, jejunostomy, ileostomy, colostomy or other surgical procedures. The mucosal barrier of the digestive tract may also be breached by ischemia or reperfusion injury. The mucosal barrier of the digestive tract may also be breached by damage caused by cancer chemotherapy, cancer radiation therapy, or high dose radiation exposure outside of a therapeutic setting.

The mucosal barrier of the digestive tract may be breached or compromised through gross inflammation and /or ulceration, including but not limited to periodontal disease, aphthous stomatitis, bacterial, viral, fungal or parasitic infections of the digestive tract, peptic ulcers, ulcers associated with stress or H. pylori infection, damage caused by esophageal reflux, inflammatory bowel disease, damage caused by cancer of the digestive tract, food intolerance, including celiac disease, or ulcers induced by non-steroidal anti-inflammatory drugs (NSAIDs) or other ingested or systemically delivered drugs.

The breach in or compromise of the mucosal barrier of the digestive tract may be one that has been described clinically but where the biological basis for the barrier defect is not well understood, including but not limited to the loss of gut barrier function associated with external burns, trauma, sepsis or shock, irritable bowel syndrome, diabetes (in particular type I diabetes), atopic dermatitis, patients suffering from autoimmune disorders, including ankylosing spondylitis, Sjogren's syndrome, congestive heart failure, or multiple sclerosis. Infections with pathogens may also cause specific disruptions of barrier function.

In some diseases or disorders to which this invention may be applied, altered barrier permeability may be present prior to the development of frank inflammation and/or ulceration and antibodies may be applied at the time of altered barrier permeability as well as during the time of inflammation and ulceration. Diseases and disorders which include increased permeability prior to inflammation include but are not limited to mucositis induced by chemotherapy or radiation therapy, by exposure to high levels of non-therapeutic radiation, inflammatory bowel disease and celiac disease.

In some diseases or disorders to which this invention may be applied, altered barrier permeability may be present at discrete portions of the digestive tract while frank inflammation and/or ulceration is present at other portions of the digestive tract. Diseases and disorders which include physically separated regions of increased permeability and inflammation or ulceration include but are not limited to Crohn's disease and ulcerative colitis. Antibodies of this invention may be used to access the regions of altered permeability as well as the regions of frank inflammation and ulceration.

This invention includes the use of antibodies as therapeutics that are designed to address the underlying cause of the barrier defect. Such antibodies may be directed at biological targets that enhance wound healing, that alter the function of tight junctions, or at other targets known now or in the future that affect permeability. Suitable targets may include but are not limited to occludin, claudins, junctional adhesion molecule, ZO-1, E-cadherin, coxackie adenovirus receptor and serine proteases such as elastase that are involved in the release of claudins.

This invention includes the use of antibodies as pharmaceutical agents that are designed to bind to biological targets unrelated to the underlying cause of the barrier defect. Such antibodies may be used to treat or prevent diseases and disorders relating to the same disease state that caused the barrier defect. Such antibodies may be used to treat or prevent diseases and disorders unrelated to the disease state that caused the barrier defect.

For disorders of the oral cavity, the antibodies of the invention can be delivered in a mouthwash, rinse, paste, gel, or other suitable formulation. Antibodies of the invention can be delivered using formulations designed to increase the contact between the active antibody and the mucosal surface, such as buccal patches, buccal tape, mucoadhesive films, sublingual tablets, lozenges, wafers, chewable tablets, quick or fast dissolving tablets, effervescent tablets, or a buccal or sublingual solid. For disorders of the digestive tract, antibody can be delivered by oral ingestion in the form of a capsule, tablet, liquid formulation or similar form designed to introduce drug to the digestive tract. Alternatively, antibody may be administered by suppository or enema for delivery to the lower digestive tract. Such formulations are well known to those skilled in the art.

The terms "antibody" or "antibodies" as used herein refer to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding to a target receptor. An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by degradation with various peptidases that are able to compete with the intact antibody for specific binding, unless otherwise specified herein. Thus, for example, pepsin digests an antibody below the disulfide linkages in the hinge region to produce F(ab)'₂, a dimer of Fab which itself is a light chain joined to V_{H-CH}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (see *Fundamental Immunology* (Paul ed., 3d ed. 1993). While various antibody fragments are defined in terms of the degradation of an intact antibody, one of skill will appreciate that such fragments may be synthesized *de novo* either chemically or by using recombinant DNA methodology. Thus, the term "antibody", as used herein, also includes antibody fragments either produced by the modification of whole antibodies, or those synthesized *de novo* using chemical or recombinant DNA methodologies (e.g., single chain Fv, complementarity determining region (CDR) fragments, or polypeptides that contain at least a portion of an immunoglobulin that is sufficient to confer specific receptor binding to the polypeptide) or those identified using phage display libraries (see, e.g., McCafferty et al., Nature 348: 552-554 (1990)).

The terms "monoclonal antibody" or "monoclonal antibodies" as used herein refer to a preparation of antibodies of single molecular composition. A monoclonal antibody composition displays a single binding specificity and affinity for a particular epitope of a target receptor.

An "epitope" is the portion of a molecule that is bound by an antibody. An epitope can comprise non-contiguous portions of the molecule (e.g., in a polypeptide, amino acid residues that are not contiguous in the polypeptide's primary sequence but that, in the context of the polypeptide's tertiary and quaternary structure, are near enough to each other to be bound by an antibody).

The term "polyclonal antibody" as used herein refers to a composition of different antibody molecules which is capable of binding to or reacting with several different specific antigenic determinants on the same or on different antigens. The variability in antigen specificity of a polyclonal antibody is located in the variable regions of the individual antibodies constituting the polyclonal antibody, in particular in the complementarity determining regions (CDR)1, CDR2 and CDR3 regions. Preferably, the polyclonal antibody is prepared by immunization of an animal with the target antigen or portions thereof as specified below. Alternatively, the polyclonal antibody may be prepared by mixing multiple monoclonal antibodies (e.g. Nowakowski, A. et al., 2002. Proc Natl Acad Sci USA 99, 11346-11350 and U.S. Pat. No. 5,126,130) having desired specificity to a target receptor.

Polyclonal antibody preparations isolated from the blood, milk, colostrum or eggs of immunized animals typically include antibodies that are not specific for the immunogen in addition to antibodies specific for the target antigen. Antibodies specific for the target antigen may be purified from the polyclonal antibody preparation or the polyclonal antibody preparation may be used without further purification. Thus, the term "polyclonal antibody" as used herein refers both to antibody preparations in which the antibody specific for the target antigen has been enriched and to preparations that are not purified. Numerous techniques are known to those in the art for enriching polyclonal antibodies for antibodies to specific targets. Recently a technology for recombinant production of highly specific polyclonal antibodies suitable for prophylactic and therapeutic administration has been developed (WO 2004/061104). The recombinant polyclonal antibody (rpAb) can be purified from a production bioreactor as a single preparation without separate handling, manufacturing, purification, or characterization of the individual members constituting the recombinant polyclonal protein.

In one embodiment, the antibody is a polyclonal antibody derived from milk or colostrum. In one embodiment, the polyclonal antibody is derived from the milk or colostrum of a ruminant such as a cow, goat, sheep, camel or water buffalo. In another embodiment, the antibody is isolated from the milk or colostrum of a human. In a preferred embodiment, the polyclonal antibody is isolated from the milk or colostrum of a bovine, preferably an immunized cow. Bovine colostrum (early milk) is a preferred source of antibodies for this invention. In cows, antibody does not cross the placenta, and thus all passive immunity is transferred to the newborn calf through the milk. As a result, cows secrete a large bolus of antibody into the colostrum immediately after parturition and approximately 50% of the protein in colostrum is immunoglobulin. In the first 4 hours after birth, immunoglobulin concentrations of 50 mg/ml are typically found in the colostrum (Butler and Kehrli, 2005, Mucosal Immunology, 1763-1793), dropping to 25 - 30 mg/ml 24 hours later (Ontsouka et al., 2003, J Dairy Sci, 86, 2005-11). Colostrum and milk are a uniquely safe source of polyclonal antibody for oral delivery. There is already extensive human exposure to bovine immunoglobulin, as regular milk contains 1.5 g/L IgG.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. See, e.g., U.S. Pat. No. 4,816,567 and Morrison, 1985, Science 229:1202-07.

The invention further contemplates the use of molecules intended to mimic antibodies, such as aptamers, nanobodies and fibronectin-based antibody mimics. The invention also contemplates the use of "fusion proteins" in which a portion of an antibody molecule is fused to the ligand for the target receptor and thereby made specific for the target receptor. In another aspect, the present invention provides a derivative of an antibody specific for a target. The derivatized antibody can comprise any molecule or substance that imparts a desired property to the antibody, such as increased half-life in a particular use. The derivatized antibody can comprise, for example, a detectable (or labeling) moiety (e.g., a radioactive, colorimetric, antigenic or enzymatic molecule, a detectable bead (such as a magnetic or electrodense (e.g., gold bead), or a molecule that binds to another molecule (e.g., biotin or streptavidin)), a therapeutic or diagnostic moiety (e.g., a radioactive, cytotoxic, or pharmaceutically active moiety), or a molecule that increases the suitability of the antibody for a particular use (e.g., administration to a subject, such as a human subject, or other *in vivo* or *in vitro* uses). Examples of molecules that can be used to derivatize an antibody include albumin (e.g., human serum albumin) and polyethylene glycol (PEG). Albumin-linked and PEGylated derivatives of antibodies can be prepared using techniques well known in the art. In one embodiment, the antibody is conjugated or otherwise linked to transthyretin (TTR) or a TTR variant. The TTR or TTR variant can be chemically modified with, for example, a chemical selected from the group consisting of dextran, poly(n-vinyl pyurrolidone), polyethylene glycols, propropylene glycol homopolymers, polypropylene oxide/ethylene oxide co-polymers, polyoxyethylated polyols and polyvinyl alcohols.

In one aspect, the invention provides methods of treating a patient using the therapeutic compositions of the invention. The term "patient" as used herein refers to an animal. Preferably the animal is a mammal. More preferably the mammal is a human. A "patient" also refers to, for example, dogs, cats, horses, cows, pigs, guinea pigs, fish, birds and the like. Thus, the compositions and methods of the invention are equally suitable for veterinary treatments. In one embodiment of the invention, antibodies are used to treat diseases or disorders of companion animals, work animals or animals raised for food. In one embodiment of the invention, stabilized antibodies are used to provide passive immunity to newborn animals, preferably to cows, horses, sheep or swine.

The terms "treatment" "treat" and "treating" encompasses alleviation, cure or prevention of at least one symptom or other aspect of a disorder, disease, illness or other condition (collectively referred to herein as a "condition"), or reduction of severity of the condition, and the like. A composition of the invention need not affect a complete cure, or eradicate every symptom or manifestation of a disease, to constitute a viable therapeutic agent. As is recognized in the pertinent field, drugs employed as therapeutic agents may reduce the severity of a given disease state, but need not abolish every manifestation of the disease to be regarded as useful therapeutic agents. Similarly, a prophylactically administered treatment need not be completely effective in preventing the onset of a condition in order to constitute a viable prophylactic agent. Simply reducing the impact of a disease (for example, by reducing the number or severity of its symptoms, or by increasing the effectiveness of another treatment, or by producing another beneficial effect), or reducing the likelihood that the disease will occur or worsen in a subject, is sufficient. In one embodiment, an indication that a therapeutically effective amount of a composition has been administered to the patient is a sustained improvement over baseline of an indicator that reflects the severity of the particular disorder.

The pharmaceutical compositions of the present invention comprise a therapeutically effective amount of an antibody of the present invention formulated together with one or more pharmaceutically acceptable carriers or excipients. By a "therapeutically effective amount" of an antibody of the invention is meant an amount of the composition which confers a therapeutic effect on the treated subject, at a reasonable benefit/risk ratio applicable to any medical treatment. The therapeutic effect is sufficient to "treat" the patient as that term is used herein.

As used herein, the term "pharmaceutically acceptable carrier or excipient" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol; esters such as ethyl oleate and ethyl laurate; agar; buffering agents such as magnesium hydroxide and aluminun hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Compositions for rectal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and therefore melt in the rectum or vaginal cavity and release the active compound. In one embodiment, compositions for rectal administration are in the form of an enema.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

Although stabilized antibodies have enhanced stability to gastric degradation, it may be desirable under some conditions to provide additional levels of protection against gastric degradation. If this is desired, there are many options for enteric coating (see for example U.S. patents 4,330,338 and 4,518,433). In one embodiment, enteric coatings take advantage of the post-gastric change in pH to dissolve a film coating and release the active ingredient. Coatings and formulations have been developed to deliver protein therapeutics to the small intestine and these approaches could be adapted for the delivery of an antibody of the invention. For example, an enteric-coated form of insulin has been developed for oral delivery (Toorisaka et al., 2005, J Control Release, 107, 91-6).

In addition, the solid dosage forms of tablets, dragees, capsules, pills, and granules can be prepared with other coatings and shells well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Effective doses will vary depending on route of administration, as well as the possibility of co-usage with other agents. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the timing of delivery of the compound relative to food intake; the duration of the treatment; drugs used in combination or contemporaneously with the specific compound employed; and like factors well known in the medical arts.

In accordance with the invention, routes of administration include oral administration via catheter or feeding tube.

Particular embodiments of the present invention involve administering a pharmaceutical composition comprising an antibody of the invention at a dosage of from about 1 mg per day to about 1 g/day, more preferably from about 10 mg/day to about 500 mg/day, and most preferably from about 20 mg/day to about 100 mg/day, to a subject. In one embodiment, a polyclonal antibody preparation is administered at a dosage of antibody from about 100 mg to about 50 g/day, more preferably from about 500 mg/day to about 10 g/day, and most preferably from about 1 g/day to about 5 g/day, to a subject, wherein the polyclonal antibody preparation has not been enriched for antibodies specific for the target antigen.

Treatment regimens include administering an antibody composition of the invention one time per day, two times per day, or three or more times per day, to treat a medical disorder disclosed herein. In one embodiment, an antibody composition of the invention is administered four times per day, 6 times per day or 8 times per day to treat a medical disorder disclosed herein. In one embodiment, an antibody composition of the invention is administered one time per week, two times per week, or three or more times per week, to treat a medical disorder disclosed herein.

The methods and compositions of the invention include the use of an antibody of the invention in combination with one or more additional therapeutic agents useful in treating the condition with which the patient is afflicted. Examples of such agents include both proteinaceous and non-proteinaceous drugs. When multiple therapeutics are co-administered, dosages may be adjusted accordingly, as is recognized in the pertinent art. "Co-administration" and combination therapy are not limited to simultaneous administration, but also include treatment regimens in which an antibody of the invention is administered at least once during a course of treatment that involves administering at least one other therapeutic agent to the patient.

In one preferred embodiment, the invention comprises compositions and methods for treating inflammation, and particularly inflammatory bowel disease using antibodies specific for TNF, and preferably milk-derived, polyclonal anti-TNF antibodies while maintaining peak anti-TNF antibody serum concentrations below 1 µg/ml, preferably below 300 ng/ml, preferably below 100 ng/ml. In another preferred embodiment the invention comprises compositions methods for treating mucositis and particularly oral mucositis using milk-derived polyclonal, anti-TNF antibodies while maintaining peak anti-TNF antibody serum concentrations below 1 µg/ml. Other preferred embodiments directed to disorders of the digestive tract that are capable of treatment using antibodies of the invention that are specific for targets that are accessible to such antibodies when administered topically to the digestive tract while maintaining antibody serum concentrations below 1 µg/ml are also described herein.

The following examples are provided for the purpose of illustrating specific embodiments or features of the invention and are not intended to limit its scope.

### Examples

### Example 1. Analysis of bovine anti-TNF antibody

Bovine polyclonal anti-TNF antibody (AVX-470) was generated by immunizing cows with murine TNF and collecting the colostrum post-parturition. Immunoglobulin was purified from colostral whey by ammonium sulfate precipitation; the preparation was found to be 70% pure by SDS-PAGE. Control bovine immunoglobulin from the colostrum of cows that had been immunized with gluten was purified in parallel. Protein concentration of the semi-purified immunoglobulin preparations were determined using the BCA assay.

The TNF-neutralizing activity of AVX-470 was assayed using the standard L929 assay. Because the first functional experiments carried out with AVX-470 were to be performed in hamsters, the ability to neutralize hamster TNF was assessed. Hamster splenocytes were stimulated with LPS as 2 ng/ml for 24 hr and the supernatant used as a source of hamster TNF. Varying concentrations of AVX-470, control bovine anti-gluten antibody or purified rabbit anti-TNF antibody (Biovision) were incubated for 2 hr at 37°C with a 1:100 dilution of the hamster LPS supernatant (hereinafter referred to as hamster TNF). The hamster TNF-antibody combination was transferred, along with 1 ug/ml actinomycin D to L929 cells (3.5 x 10⁴ cells per well) that had been plated overnight. Cultures were incubated overnight and cell proliferation was measured using WST. All assays were carried out in triplicate.

As shown in Table I, hamster TNF inhibited the proliferation of L929 cells. This inhibition was blocked in a dose-dependent fashion by the purified rabbit anti-TNF antibody and by AVX-470, but not by the control bovine anti-gluten antibody. Half-maximal inhibition was achieved by 0.5 ug/ml purified anti-TNF antibody and by 200 ug/ml AVX-470. Based on these data, it is estimated that 0.25% of the antibody in AVX-470 is specific for TNF.

| **Table I** | | | |
|---|---|---|---|
| | **cone (µg/ml)** | **Ave ± SEM** | **% inhibition** |
| Medium | | 0.842 ± 0.076 | |
| Hamster TNF | | 0.301 ± 0.001 | |
| Rabbit anti-TNF | 0.50 | 0.602 ± 0.016 | 56% |
| | 0.17 | 0.367 ± 0.010 | 12% |
| | 0.06 | 0.317 ± 0.002 | 3% |
| AVX-470 | 336.00 | 0.710 ± 0.019 | 76% |
| | 112.00 | 0.419 ± 0.022 | 22% |
| | 37.33 | 0.367 ± 0.008 | 12% |
| Anti-gluten | 238.00 | 0.306 ± 0.003 | 1% |
| | 79.33 | 0.326 ± 0.003 | 5% |
| | 26.44 | 0.335 ± 0.001 | 6% |

### Example 2. Treatment of TNBS-induced colitis with oral anti-TNF antibody

Polyclonal bovine anti-TNF antibody (AVX-470) was isolated from the colostrum of cows that had been immunized with murine TNF. Colostral whey was produced by defatting the colostrum and precipitating casein by incubation at pH 4.6. Whey from immunized cows was pooled, and purified by thiophilic adsorbent chromatography. Control antibody was purified from the colostrum of a non-immunized animal in parallel.

C57BL/6 mice ((8-9 weeks old) Charles River Laboratories, Wilmington, MA) were administered 0.1 mL TNBS (trinitrobenzene sulfonate) (4 mg) in 50% ethanol intrarectally. The TNBS model is a well-accepted model of inflammatory bowel disease. Control animals were dosed with ethanol alone. Twelve animals were used in TNBS-treated group and eight animals in each of the other groups. Animals were dosed with AVX-470 (5 mg, 1.5 mg or 0.5 mg), control bovine antibody (1.5 mg) or saline twice per day by oral gavage in 0.1 ml. Antibody was administered from day -1 to day 3.

Each mouse was analyzed using video endoscopy, under isoflurane anesthesia, on day 5 just prior to the animals being sacrificed. During each endoscopic procedure still images as well as video were recorded to evaluate the extent of colitis and the response to treatment. Colitis severity was scored by a blinded observer using a 0-4 scale (0= normal; 1= loss of vascularity; 2= loss of vascularity and friability; 3= friability and erosions; 4= ulcerations and bleeding). Differences between groups were analyzed using the Student's T-test.

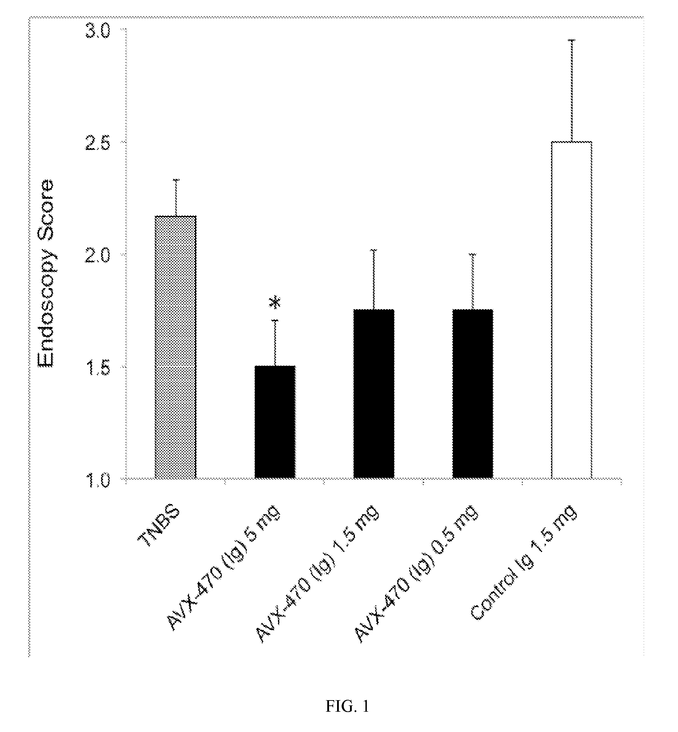

As shown in Figure 1, oral administration of AVX-470 reduced the severity of colitis in this well accepted model of inflammatory bowel disease.

### Example 3. Serum antibody levels following treatment of TNBS-induced colitis with oral anti-TNF antibody

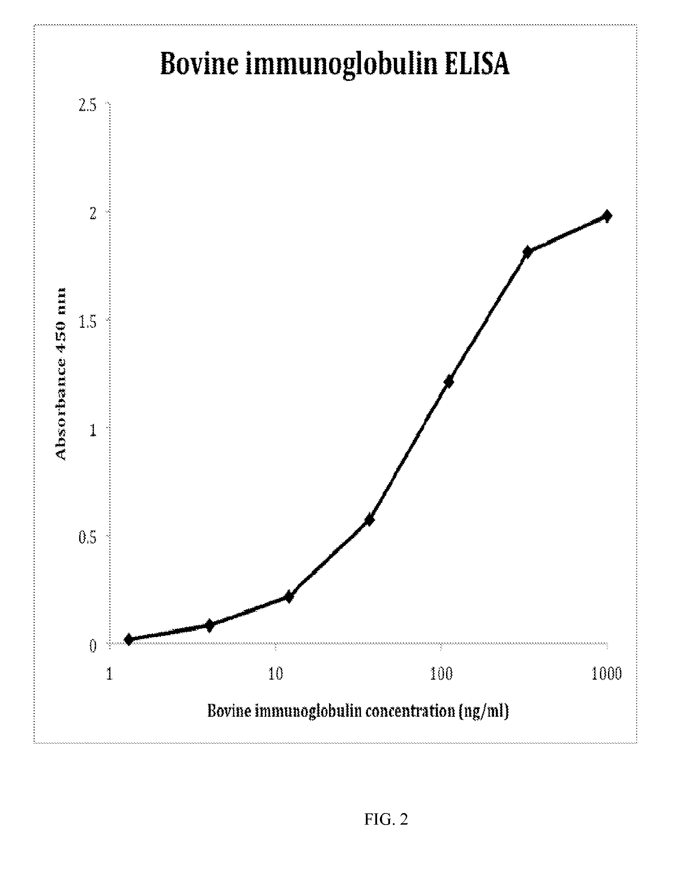

Following sacrifice of the mice in Example 2, blood samples were collected by cardiac puncture and serum was prepared and frozen. Serum from each individual mouse was assayed for the presence of bovine immunoglobulin by ELISA. Plates were coated with sheep anti-bovine IgG (h+1) antibody at 5 ug/ml (Bethyl Laboratories, Montgomery, TX). Serial dilutions of mouse serum (3-fold dilutions, starting at 1:100 dilution) were analyzed in duplicate. Antibody was detected with HRP-labeled sheep anti-bovine IgG (h+1) antibody at 10 ng/ml and TMB substrate (SigmaAldrich, St. Louis, MO). A standard curve using bovine reference serum (Bethyl Laboratories) was run on every ELISA plate. As shown in Figure 2, the assay readily detects concentrations of bovine immunoglobulin greater than 10 ng/ml.

Serum from all mice was analyzed in the ELISA. 31 of the 32 mice dosed with bovine antibody (AVX-470 or control antibody) did not have detectable bovine immunoglobulin in the serum at day 5. The limit of detection in the ELISA was 10 ng/ml and serum was assayed at 1:100 dilution. Therefore, the serum samples contained < 1 ug/ml bovine immunoglobulin.

Very high levels of bovine immunoglobulin were detected in the serum of mouse #23 (0.95 mg/ml). This mouse was in the group treated with the highest dose of AVX-470 (5 mg). The serum volume of a mouse is approximately 1 ml, and these data would suggest that 20% of the applied dose was present in the serum. However, given that the other 7 mice in this group had serum levels of bovine immunoglobulin < 1 ug/ml (>1000-fold lower), it is likely that this result reflects contamination of the sample. It should be noted that mouse #23 had the highest endoscopy score in the high dose treated group.

The data shown in Example 1 indicate that <1% of the immunoglobulin in AVX-470 is specific for TNF. Therefore, the serum concentration of TNF-specific antibody is less than 10 ng/ml in this experiment where a significant clinical effect was seen. Clinically effective serum concentrations for injected anti-TNF antibodies are 0.8-1.4 ug/ml (Tracey et al., 2008, Pharmacol Ther, 117, 244-79).

### Example 4. Treatment of oral mucositis with topical anti-TNF antibody

Polyclonal bovine anti-TNF antibody (AVX-470) was isolated from the colostrum of cows that had been immunized with murine TNF. Colostral whey was produced by defatting the colostrum and precipitating casein by incubation at pH 4.6. Whey from immunized cows was pooled, and purified by ammonium sulfate precipitation. Control antibody was purified in parallel from the colostrum of cows that had been immunized with gluten.

Syrian Golden hamsters (8 hamsters per group) were anesthetized and the left buccal pouch was everted, fixed and isolated using a lead shield. A single dose of radiation (40 Gy/dose) was administered to all animals on day 0 at a rate of 2.0 Gy/minute. Radiation was generated with a 16- kilovolt potential (15-ma) source at a focal distance of 50 cm, hardened with a 0.35 mm Cu filtration system. In this model, mucositis severity reaches a maximum on day 14, and begins to heal by day 18. Starting at day 0, immediately after radiation, hamsters were administered 1 mg of AVX-470 in saline buffer in the left buccal cheek pouch twice a day throughout the 28 day study.

Two control groups were included - a saline group and a group dosed with 7 mg bovine colostral anti-gluten antibody. Mucositis severity was evaluated every other day starting on day 6 and continuing through day 28. Animals were anesthetized and the left cheek pouch everted and photographed. At the end of the study, the images were randomized and scored in an independent manner by 2 scorers who were blinded as to the identifiers for each image.

Mucositis was scored visually by comparison to a validated photographic scale. Score of 0: Pouch completely healthy. No erythema or vasodilation; Score of 1: Light to severe erythema and vasodilation. No erosion of mucosa; Score of 2: Severe erythema and vasodilation. Erosion of superfcial aspects of mucosa leaving denuded areas. Decreased stippling of mucosa; Score of 3: Formation of off-white ulcers in one or more places. Ulcers may have a yellow/gray color due to pseudomembrane. Cumulative size of ulcers equals ∼¼ of the pouch. Severe erythema and vasodilation; Score of 4: Cumulative size of ulcers equals about ½ of the pouch. Loss of pliability. Severe erythema and vasodilation; Score of 5: Virtually all of pouch is ulcerated. Loss of pliability (pouch can only partially be extracted from mouth).

The difference in mucositis severity between groups was assessed by calculating the number of days each animal presented with an ulcer (i.e. a score of 3 or higher). Ulceration is the point in the development of the disease where the physical integrity of the oral mucosa is breached and is a clinically significant endpoint. As shown in Table II, animals treated with 1 mg AVX-470 had a 26% reduction in the number of animal days with grade 3 or greater mucositis. Treatment with anti-gluten antibody had no effect on the disease outcome.

| **Table II** | | | |
|---|---|---|---|
| **Group** | **Days ≥ 3** | **% of days ≥ 3** | **% Reduction** |
| Saline | 70 | 36.5 | |
| AVX-470 | 52 | 27.1 | 26% |
| Anti-gluten | 68 | 35.4 | 3% |

### Example 5. Serum antibody levels following treatment of oral mucositis with topical anti-TNF antibody.

Following sacrifice of the hamsters in Example 4, blood samples were collected by cardiac puncture and serum was prepared and frozen. Serum from each individual hamster was assayed for the presence of bovine immunoglobulin by ELISA. Plates were coated with sheep anti-bovine IgG (h+1) antibody at 5 ug/ml (Bethyl Laboratories, Montgomery, TX). Serial dilutions of mouse serum (3-fold dilutions, starting at 1:100 dilution) were analyzed in duplicate. Antibody was detected with HRP-labcled sheep anti-bovine IgG (h+1) antibody at 10 ng/ml and TMB substrate (SigmaAldrich, St. Louis, MO). A standard curve using bovine reference serum (Bethyl Laboratories) was run on every ELISA plate.

Serum from all hamsters was analyzed in the ELISA. None of the hamsters dosed with bovine antibody (AVX-470 or control anti-gluten antibody) had detectable bovine immunoglobulin in the serum at day 28. The limit of detection in the ELISA was 10 ng/ml and serum was assayed at 1:100 dilution. Therefore, the serum samples contained < 1 ug/ml bovine immunoglobulin.

### Example 6. Bovine antibody detected in local tissue by immunohistochemistry following irradiation of the cheek pouch

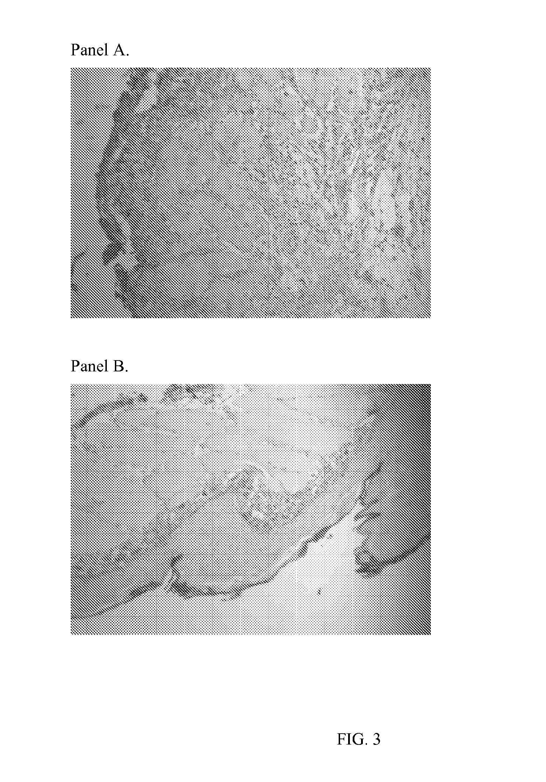

Syrian Golden hamsters received 40 Gy irradiation to the left buccal cheek pouch as in Example 4. Twelve days post-irradiation, animals received a single dose of 1 mg AVX-470 in both cheek pouches. One hour after dosing, animals were sacrificed and cheek pouches excised, fixed in formalin, embedded in paraffin and sectioned. Sections were stained for the presence of bovine immunoglobulin using the Vectastain Elite ABC kit from Vector Laboratories. The kit is designed for recognition of goat IgG, but cross-reacts with bovine immunoglobulin. As shown in Figure 3, bovine immunoglobulin could be readily detected throughout the submucosal space in the left buccal cheek pouch (panel A), but was only found on the exterior face of the non-irradiated right cheek pouch (panel B).

### Example 7. Treatment of GI Acute Radiation Syndrome with oral anti-TNF antibody

Polyclonal bovine anti-TNF antibody (AVX-470) was isolated from the colostrum of cows that had been immunized with murine TNF. Colostral whey was produced by defatting the colostrum and precipitating casein by incubation at pH 4.6. Whey from immunized cows was pooled, and purified by thiophilic adsorbent chromatography.

C3H/HeNcrl mice were administered a single dose of whole body radiation (8 Gy/dose) on Day 0. Radiation was generated with a 160 kilovolt potential (15-ma) source at a focal distance of 50 cm, hardened with a 0.35 mm Al filtration system using a Kimtron Polaris II radiation source. Irradiation targeted the total body at a rate of < 100 cGy/minute. Animals were dosed twice per day with saline or 6 mg AVX-470. The first dose was given 10 minutes after irradiation and the animals were dosed through day 10. Three animals in each group were sacrificed on days 1 and 7 and used for the experiment described in Example 8. The survival of the remaining 16 animals per group was monitored.

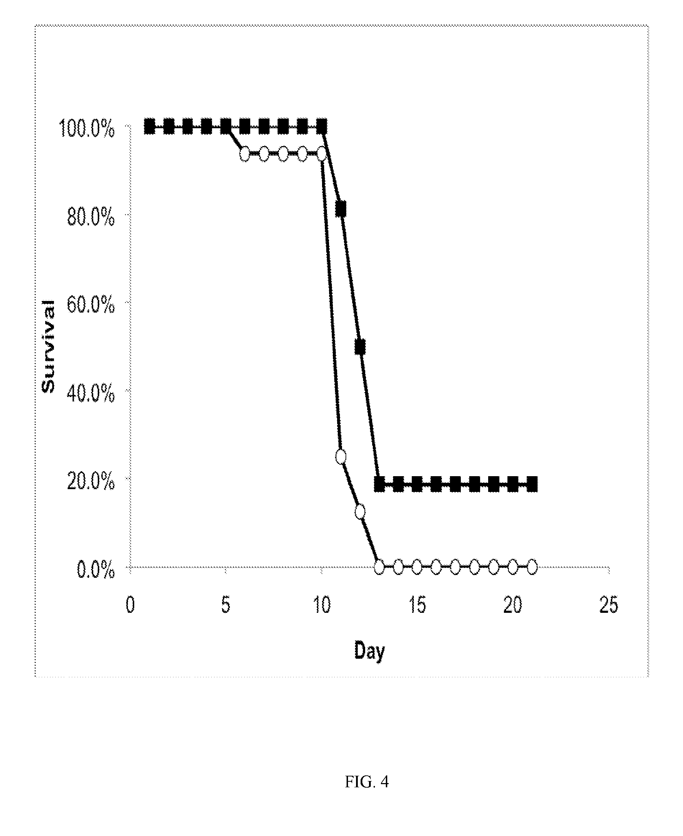

As shown in Figure 4, the group treated with AVX-470 (closed squares) showed a statistically significant improvement in survival in a Kaplan-Meier Log rank test (p=0.002), with 3 mice surviving to the end of the study compared to none in the control saline-treated group (open circles).

### Example 8. Serum antibody levels following treatment of GI ARS with oral anti-TNF antibody

Following sacrifice of the mice in Example 7 on days 1 and 7, blood samples were collected by cardiac puncture and serum was prepared and frozen. Serum from each individual mouse was assayed for the presence of bovine immunoglobulin by ELISA. Plates were coated with sheep anti-bovine IgG (h+1) antibody at 5 ug/ml (Bethyl Laboratories, Montgomery, TX). Serial dilutions of mouse serum (3-fold dilutions, starting at 1:100 dilution) were analyzed in duplicate. Antibody was detected with HRP-labeled sheep anti-bovine IgG (h+1) antibody at 10 ng/ml and TMB substrate (SigmaAldrich, St. Louis, MO). A standard curve using bovine reference serum (Bethyl Laboratories) was run on every ELISA plate.

Serum from all mice was analyzed in the ELISA. None of the mice dosed with bovine antibody (AVX-470 or control antibody) had detectable bovine immunoglobulin in the serum at day 1 or day 7. The limit of detection in the ELISA was 10 ng/ml and serum was assayed at 1:100 dilution. Therefore, the serum samples contained < 1 ug/ml bovine immunoglobulin.

### Example 9. Bovine antibody detected in small intestine lamina propria by immunohistochemistry following TBI

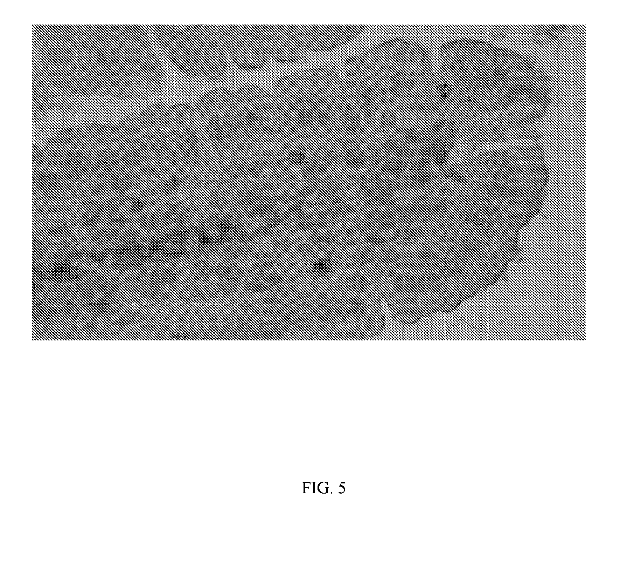

C3H/HeN mice were administered a dose of whole body irradiation of 8.55 Gy as described in Example 8. Starting immediately after irradiation, animals were dosed once per day with 10 mg AVX-470. 96 hr post-irradiation, animals were sacrificed and their small intestines removed. Sections of the jejunum were fixed in formalin, embedded in paraffin and sectioned. Sections were stained for the presence of bovine immunoglobulin using the Vectastain Elite ABC kit from Vector Laboratories. The kit is designed for recognition of goat IgG, but cross-reacts with bovine immunoglobulin. As shown in Figure 5, bovine immunoglobulin could be detected in the lamina propria.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

While this invention has been particularly shown and described with references to preferred embodiments thereof, it will be understood by those skilled in the art that various changes in form and details may be made therein without departing from the scope of the invention encompassed by the appended claims. It should also be understood that the embodiments described herein are not mutually exclusive and that features from the various embodiments may be combined in whole or in part in accordance with the invention.

## Claims

1. A pharmaceutical composition for oral administration to a patient comprising a stabilized, chimeric, anti-TNF antibody having enhanced stability to gastric degradation wherein the antibody is present in the composition in an amount effective to topically treat inflammatory bowel disease in the patient, wherein the peak level of antibody detectable in a patient's serum after administration of the antibody is less than 1 µg/ml.

2. The pharmaceutical composition of claim 1 wherein the antibody further comprises an enteric coating.

3. The pharmaceutical composition of claim 1 or claim 2 for use in a method of treating inflammatory bowel disease in a human patient in need thereof comprising orally administering the composition to the patient.
